# EUROPÄISCHE PATENTSCHRIFT

(11) **EP 1 169 313 B1**
(45) Veröffentlichungstag und Bekanntmachung des Hinweises auf die Patenterteilung: **25.06.2003**
(21) Anmeldenummer: 00920550.1
(22) Anmeldetag: 23.03.2000
(51) Int. Cl.: C07D 281/00

(54) **MIT ZUCKERRESTEN SUBSTITUIERTE 1,4-BENZOTHIAZEPIN-1,1-DIOXIDDERIVATE, VERFAHREN ZU DEREN HERSTELLUNG, DIESE VERBINDUNGEN ENTHALTENDE ARZNEIMITTEL UND DEREN VERWENDUNG**
1,4-BENZOTHIAZEPINE-1,1-DIOXIDE DERIVATIVES SUBSTITUTED BY SUGAR RADICALS, METHODS FOR THE PRODUCTION THEREOF, MEDICAMENTS CONTAINING THESE COMPOUNDS AND THE USE THEREOF
DERIVES DE 1,4-BENZOTHIAZEPIN-1,1-DIOXYDE SUBSTITUES PAR DES RESIDUS DE SUCRE, LEUR PROCEDE DE PRODUCTION, DES MEDICAMENTS CONTENANT CES COMPOSES ET LEUR UTILISATION

(30) Priorität: 09.04.1999 DE 19916108
(43) Veröffentlichungstag der Anmeldung: 09.01.2002
(73) Patentinhaber: Aventis Pharma Deutschland GmbH, 65929 Frankfurt am Main (DE)
(72) Erfinder: FRICK, Wendelin, D-65510 Hünstetten-Beuerbach (DE); GLOMBIK, Heiner, D-65719 Hofheim (DE); HEUER, Hubert, D-55270 Schwabenheim (DE); SCHÄFER, Hans-Ludwig, D-65239 Hochheim (DE)
(86) Internationale Anmeldenummer: EP0002570
(87) Internationale Veröffentlichungsnummer: WO00061568

(56) Entgegenhaltungen:
- EP-A- 0 864 582
- WO-A-96/05188

## Beschreibung

Die Erfindung betrifft substituierte 1,4-Benzothiazepin-1,1-dioxidderivate, deren physiologisch verträgliche Salze und physiologisch funktionelle Derivate.

Es sind bereits 1,4-Benzothiazepin-1,1-dioxidderivate sowie deren Verwendung zur Behandlung von Hyperlipidämie sowie Arteriosklerose und Hypercholesterinämie beschrieben worden [vgl. PCT Anmeldung Nr. PCT/GB 95/01884, Veröffentlichungs- Nr. WO 96/05188] sowie EP-A-0 864 582.

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, bereits bei einer niedrigeren Dosierung eine höhere Gallensäureauscheidung bewirken.

Die Erfindung betrifft daher Verbindungen der Formel I, 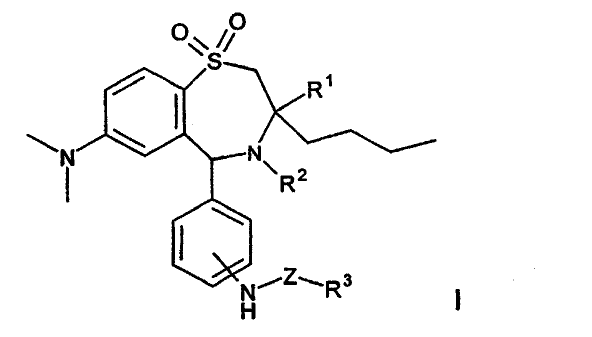 worin bedeuten
- R¹: Methyl, Ethyl, Propyl, Butyl;
- R²: H, OH;
- R³: Zuckerrest, Dizuckerrest, Trizuckerrest, Tetrazuckerrest, wobei der Zuckerrest, Dizuckerrest, Trizuckerrest oder Tetrazuckerrest gegebenenfalls ein oder mehrfach substituiert ist durch eine Zucker-Schutzgruppe;
- Z: -(C=O)ₙ-C₀-C₁₆-Alkyl-, -(C=O)ₙ-C₀-C₁₆-Alkyl-NH-, -(C=O)ₙ-C₀-C₁₆-Alkyl-O-, (C=O)ₙ-C₁-C₁₆-Alkyl-(C=O)ₘ, eine kovalente Bindung;
- n: 0 oder 1;
- m: 0 oder 1;
sowie deren pharmazeutisch verträglichen Salze und physiologisch funktionelle Derivate.

Bevorzugt sind Verbindungen der Formel I, in denen ein oder mehrere Rest(e) die folgende Bedeutung hat bzw. haben:
- R¹: Ethyl, Propyl, Butyl;
- R²: H, OH;
- R³: Zuckerrest, Dizuckerrest, wobei der Zuckerrest oder Dizuckerrest, gegebenenfalls ein oder mehrfach substituiert ist durch eine Zucker-Schutzgruppe;
- Z: -(C=O)ₙ-C₀-C₁₆-Alkyl-, -(C=O)ₙ-C₀-C₁₆-Alkyl-NH-, -(C=O)ₙ-C₀-C₁₆-Alkyl-O-,-(C=O)ₙ-C₁-C₁₆-Alkyl-(C=O)ₘ, eine kovalente Bindung;
- n: 0 oder 1;
- m: 0 oder 1;
sowie deren pharmazeutisch verträglichen Salze.

Besonders bevorzugt sind Verbindungen der Formel I, in denen ein oder mehrere Rest(e) die folgende Bedeutung hat bzw. haben:
- R¹: Ethyl, Butyl;
- R²: H, OH;
- R³: Zuckerrest, wobei der Zuckerrest gegebenenfalls ein oder mehrfach substituiert ist durch eine Zucker-Schutzgruppe;
- Z: -(C=O)-C₀-C₄-Alkyl, eine kovalente Bindung;
sowie deren pharmazeutisch verträglichen Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Gegenstand der Erfindung sind weiterhin sowohl Isomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

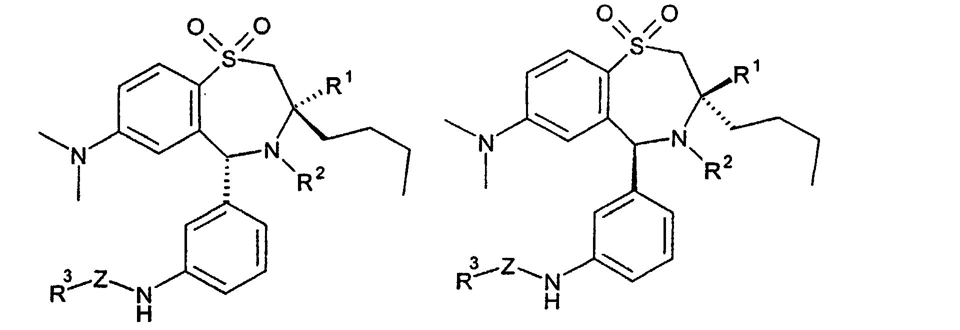

Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker, Zuckeralkohole oder Zuckersäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure.

Mit Dizucker sind Saccharide gemeint, die aus zwei Zuckereinheiten bestehen. Di-, Tri-, oder Tetrasaccharide entstehen durch acetalartige Bindung von 2 oder mehreren Zuckern. Die Bindungen können dabei in der α- oder β-Form auftreten. Beispielhaft seien genannt Laktose, Maltose und Cellobiose.

Wenn der Zucker substituiert ist, so erfolgt die Substitution bevorzugt am Wasserstoffatom einer OH-Gruppe des Zuckers.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder Isopropylidenschutzgruppen.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Die Verbindungen können gegebenenfalls auch in Kombination mit Statinen, wie z.B. Simvastatatin, Fluvastatin, Pravastatin, Cerivastatin, Lovastatin oder Atorvastin verabreicht werden. Folgende Befunde belegen die pharmakologische Wirksamkeit der erfindungsgemäßen Verbindungen.

Die biologische Prüfung der erfindungsgemäßen Verbindungen erfolgte durch den Perfusionstest. Diese Prüfung untersucht die Wirkung der erfindungsgemäßen Verbindungen auf den Gallensäurentransport im lleum. Es wurden die Diastereomerengemische der Verbindungen geprüft.

Der Perfusionstest wurde wie folgt durchgeführt:

### Experimenteller Aufbau

Männliche Wistar Ratten (Gewichtsbereich 250-350 g) wurden mit Urethan (1.5 g/kg i.p.) narkotisiert und der Gallengang wurde kanülliert mit einem Polyethylenschlauch. Acht cm proximal zur lleocöcalklappe wurde ein Einschnitt ins Ileum durchgeführt und ein Silikon-Adapter für Schläuche eingenäht. Eine zweite Inzision mit Implantation eines entsprechenden Silikon-Adapters wurde im Zökum durchgeführt. Siliconschläuche wurden an die Adapter angeschlossen, um orthograd und offen (nicht rezirkulierend) das Ileum mit Perfusionspuffer mit einer Perfusionsgeschwindigkeit von 1mL/min zu perfundieren.

Die Perfusionsschläuche wurden mit Perfusionspuffer gefüllt (137 mM NaCl, 0.9 mM CaCl₂, 0.51 mM MgCl₂, 8.1 mM Na₂HPO₄, 2.7 mM KCl, 1.47 mM KH₂PO₄) (pH 7,4), 1 % (v/v) Ethanol + 1 % DMSO. Der Perfusionspuffer enthielt die Testverbindungen in Konzentrationen wie angegeben oder das Vehikel. Der Puffer wurde auf 37°C vorgewärmt. Der Perfusionspuffer enthielt 3 mM Taurocholsäure (TCA), die wiederum mit 1000 dpm/µl ³H TCA als Marker gelabelt war.

### Studiendesgin und Auswertung der Ergebnisse

Es wurde ein experimenteller Ansatz gewählt, der die Bestimmung der Hemmung des Gallensäuretransportes am individuellen Tier erlaubte. Die Galle wurde in 10 min Intervallen über 90 min gesammelt (im Falle einer sich anschließenden Auswaschphase zur Testung der Reversibilität über einen Zeitraum bis 160 min. Die Perfusion der Vehikel enthaltenden Pufferlösung über 40 min (Prä-Testsubstanz) wurde gefolgt von einer Perfusion mit Perfusionspuffer, die die Testverbindung in der zu prüfenden Konzentration enthielt (bis 90 min)

Für die Berechnung der prozentualen Hemmung durch die Testverbindung wurden die dpms (disintegrations per min/Zerfälle pro Minute von ³H-TCA) in der Galle von 80-90 min (Ende der Perfusion mit der Testsubstanz) auf die Sammelperiode 30-40 min während der Vorphase bezogen, wenn in der Kontrollphase die Ausscheidung der ³H-TCA ihr Maximum und Plateau erreicht hatte. Es wurde die EC₅₀ (= Effective concentration 50) als die wirksame Konzentration zwischen den Hemmwerten unterschiedlicher Konzentration errechnet, die die maximale Gallensäureausscheidung um 50% hemmte.

### Ergebnisse

**Tabelle 1:**

| Verbindungen aus Beispiel | EC₅₀ Ileum-perfusion (µM) |
|---|---|
| 1 | 0,09 |
| 2 | 0,15 |
| 3 | 0,22 |
| 4 | 0,72 |
| 5 | 0,4 |
| 6 | 0,09 |
| 7 | 1,4 |
| | |
| Vergleichsbeispiel | |
| 1 | 9,8 |

Aus den Meßdaten ist abzulesen, daß die erfindungsgemäßen Verbindungen der Formel I gegenüber den im Stand der Technik beschriebenen Verbindungen eine um den Faktor 7 bis 100 bessere Wirkung aufweisen.

Die nachfolgenden Beispiele dienen zur näheren Erläuterung der Erfindung, ohne dieselbe auf in den Beispielen beschriebene Produkte und Ausführungsformen einzuschränken.

### Beispiel 1

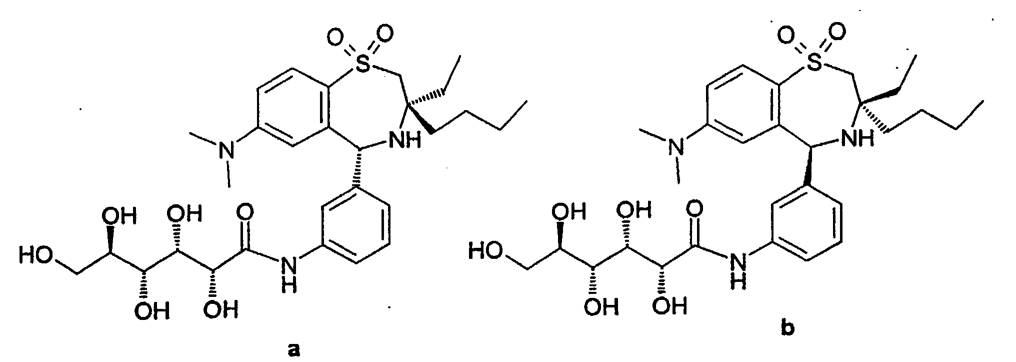

C₂₉H₄₃N₃O₈S (593.74). MS (M + H)⁺ = 594.3

### Beispiel 2

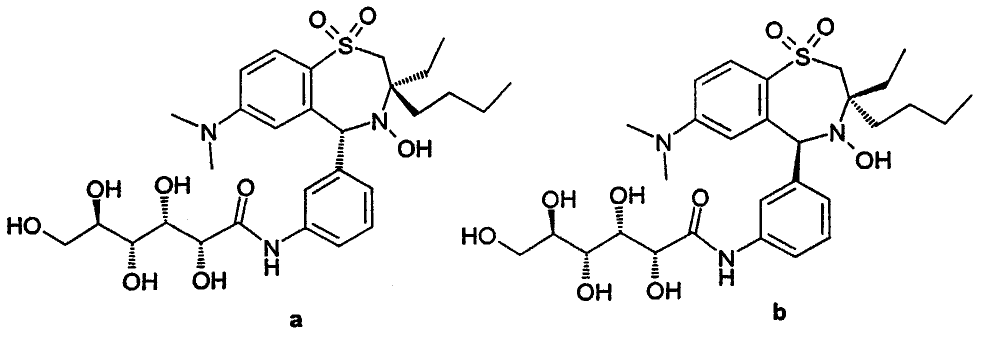

C₂₉H₄₃N₃O₉S (609.74), MS (M + H)⁺ = 610.4

### Beispiel 3

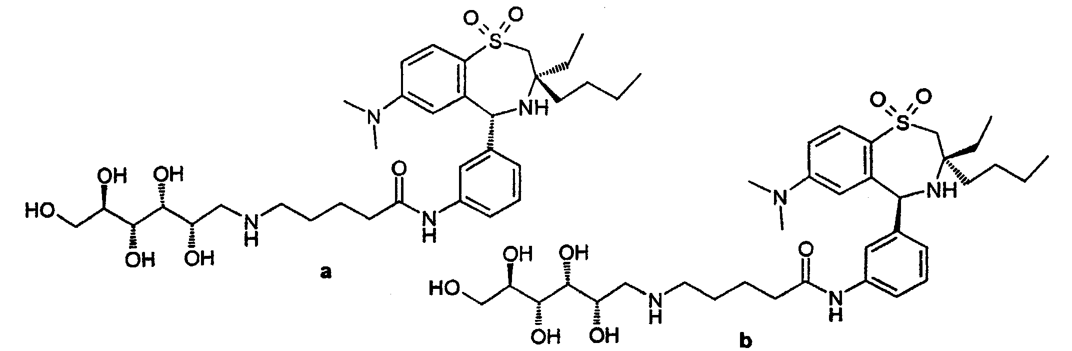

C₃₄H₅₄N₄O₈S (678.89). MS (M + H)⁺ = 679.4

### Beispiel 4

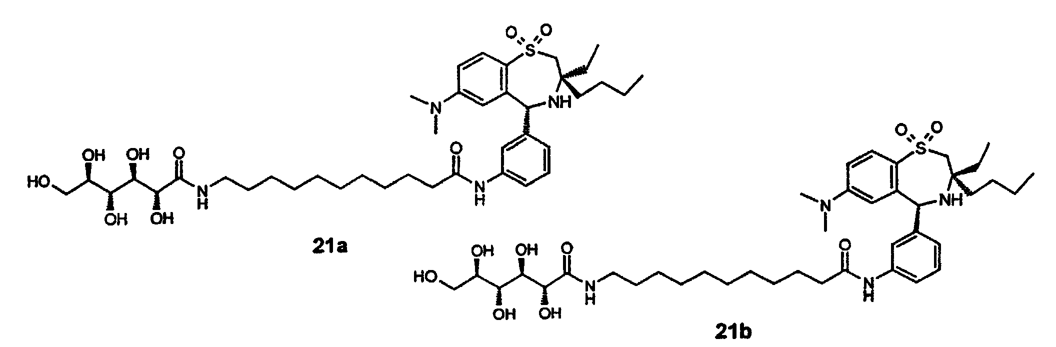

C₄₁H₆₄N₄O₉S (777.03). MS (M + H)⁺ = 777.6

### Beispiel 5

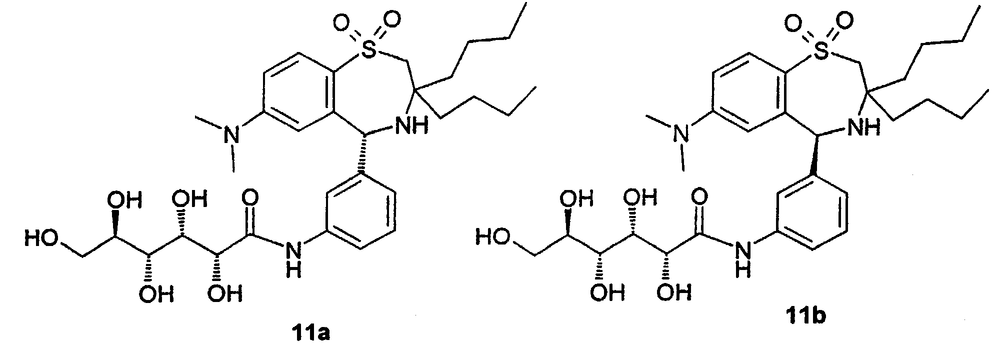

C₃₁H₄₇N₃O₈S (621.79). MS (M + H)⁺ = 622.4

### Beispiel 6

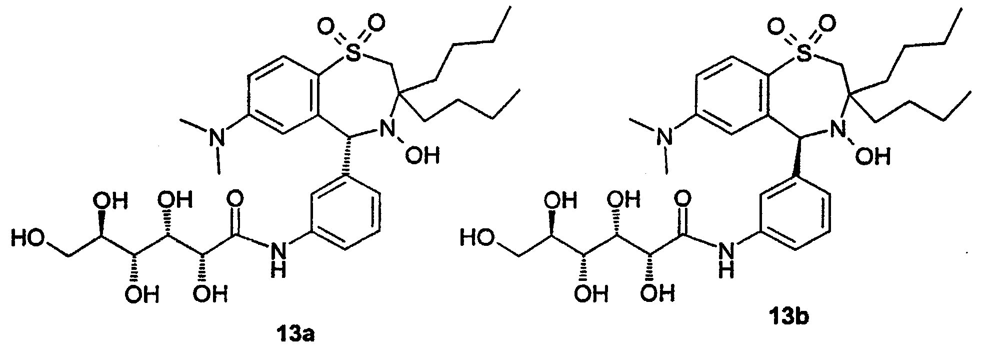

C₃₁H₄₇N₃O₉S (637.79). MS (M + H)⁺ = 638.5

### Beispiel 7

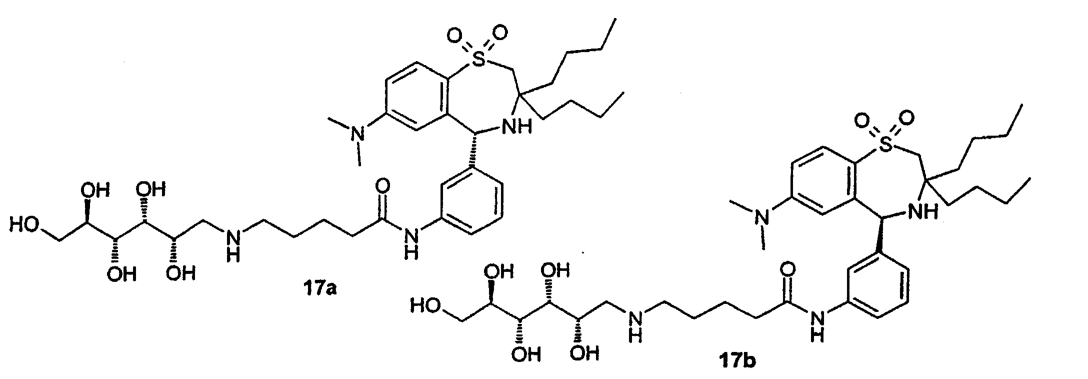

C₃₆H₅₈N₄O₈S (706.94). MS (M + H)⁺ = 707.6

Vergleichsbeispiel aus WO 96/05188 (Beispiel Nr. 20, 264W94(Glaxo Wellcome)):

### Vergleichsbeispiel 1

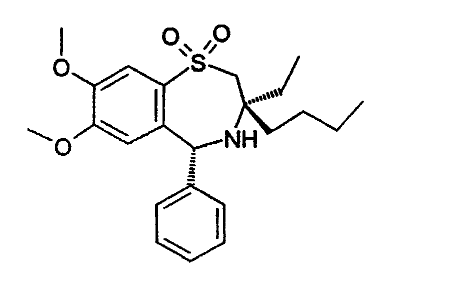

Die Beispiele wurden wie folgt hergestellt:

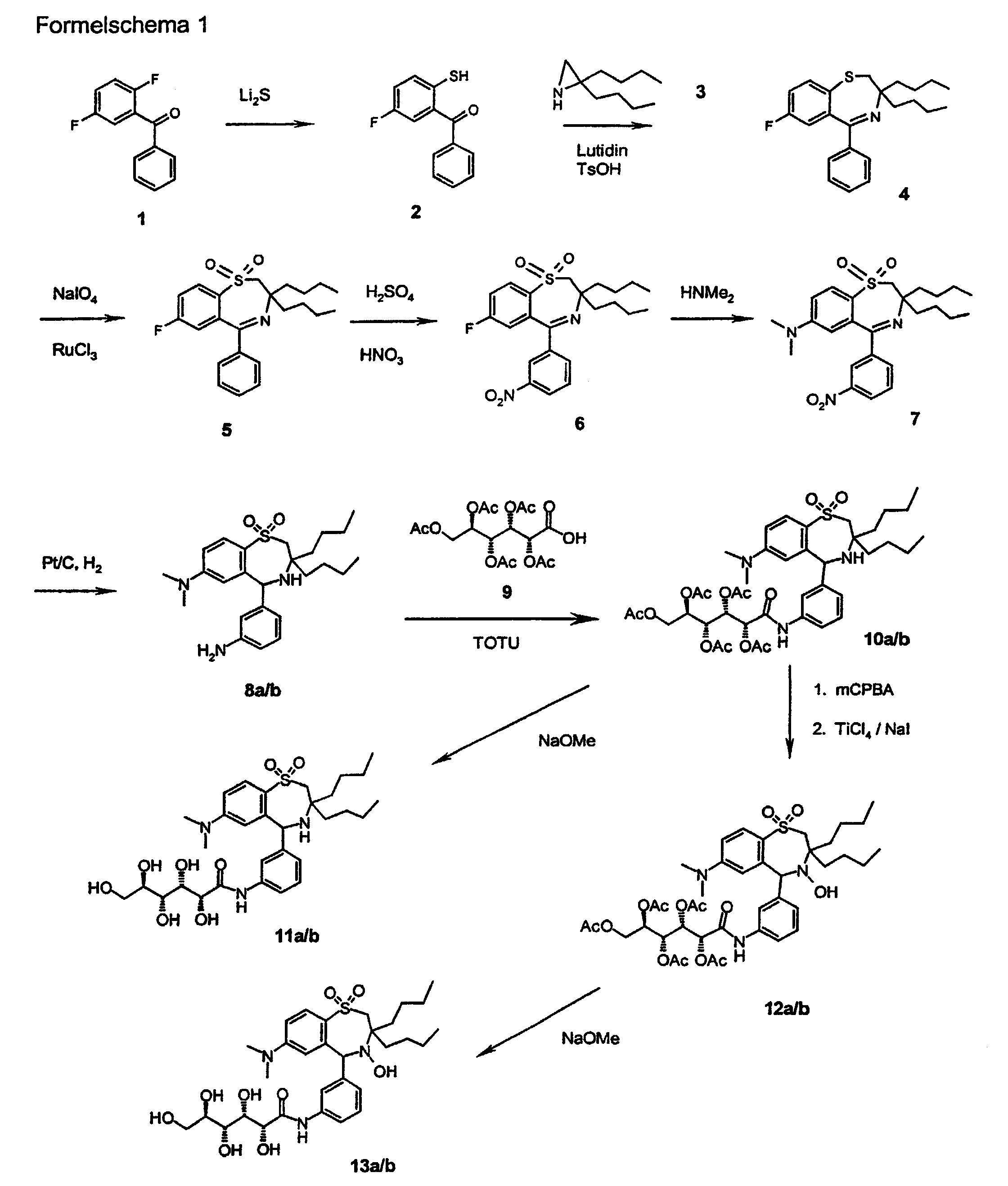

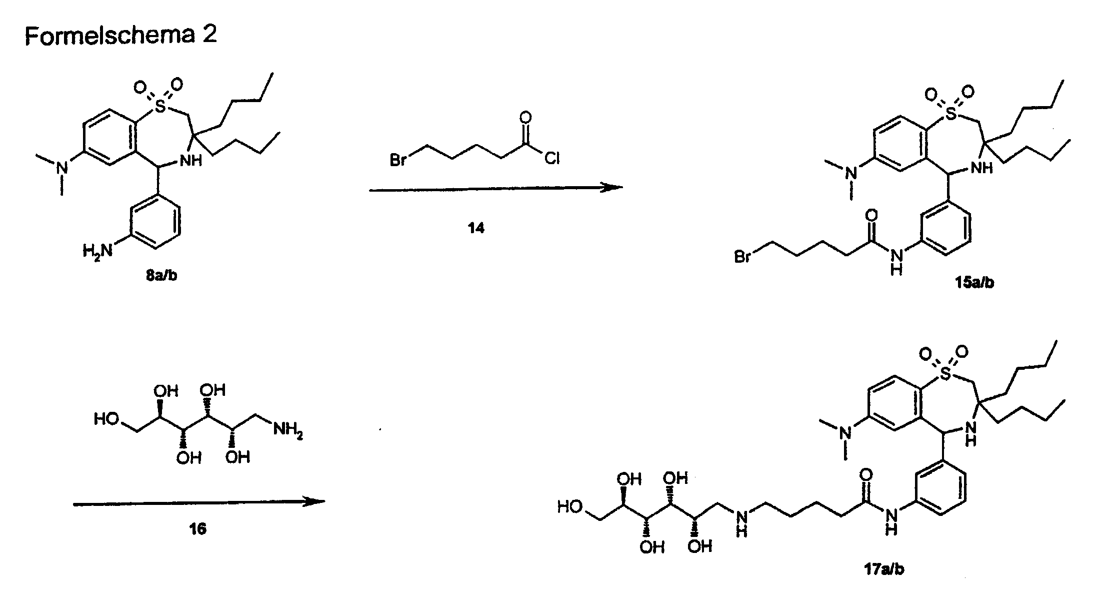

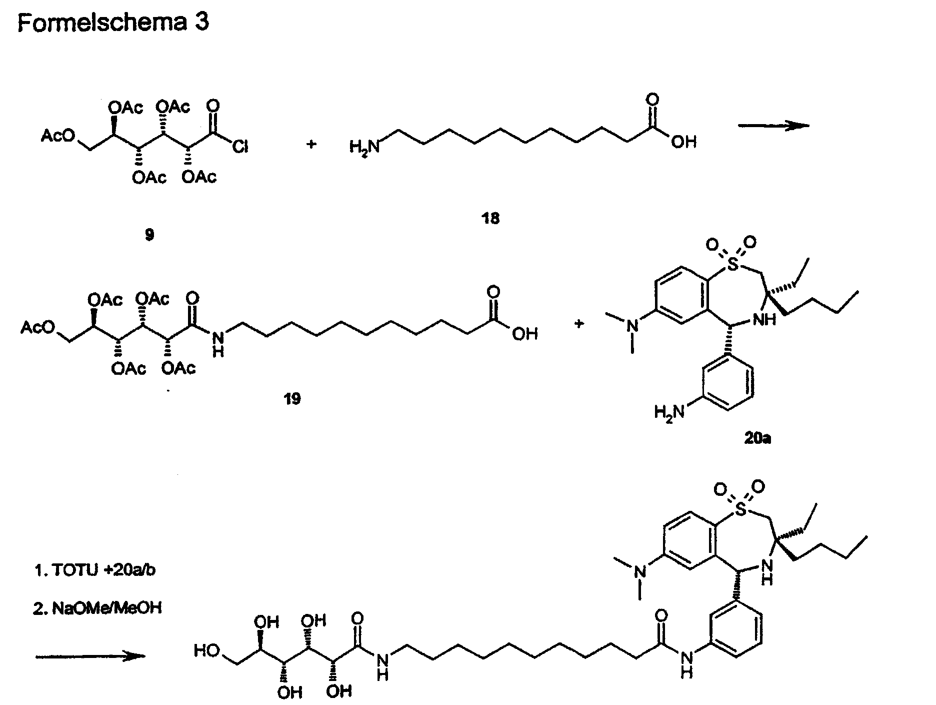

### Synthese von Verbindung 2:

20g (91.6 mmol) 2,5-Difluorbenzophenon **1** (Fluka) werden in 400 ml DMSO gelöst. Unter Argon werden 7.0g (150 mmol) Lithiumsulfid (Fluka) zugegeben. Nach drei Std. bei 120°C läßt man auf RT abkühlen. Es wird mit 200 ml 2 M HCI aq. und 500 ml Ethylacetat geschüttelt. Die organische Phase wird noch zweimal mit NaCI- Lsg. gewaschen, über MgSO₄ getrocknet, filtriert und eingeengt. Man erhält 24g Rohprodukt **2** als rötliches Öl. DC (n- Heptan/ Ethylacetat 3:1). R_{f} = 0.3, Edukt **1** R_{f} = 0.4. C₁₃H₉FOS (232.28). MS (M+H)⁺ = 233.1.

### Synthese von Verbindung 4:

7g Rohprodukt **2**, 2.5g (16 mmol) Dibuthylaziridin **3** (R. Gauthier et al., J. Organomet. Chem. 140 **(1977)** 245 - 255) und 300mg p-Toluolsulfonsäure werden in 100 ml Lutidin gelöst. Die Reaktionslösung wird drei Std. am Wasserabscheider gekocht. Danach wird eingeengt und der Rückstand mit Flashchromatographie gereinigt. Ausbeute 3.6g (61%) **4** als farbloses Öl. DC (n- Heptan/ Ethylacetat 9:1). R_{f} = 0.5. C₂₃H₂₈FNS (369.55). MS (M+H)⁺ = 370.3.

### Synthese von Verbindung 5:

3.6g (9.7 mmol) **4** und 6.0g NalO₄ werden in 100 ml Acetonitril, 50 ml Methylenchlorid und 30 ml Wasser suspendiert. Nach Zugabe von 200mg RuCl₃ läßt man 2 Std bei Raumtemperatur kräftig rühren. Die Lösung wird mit 200 ml Ethylacetat verdünnt und 2x mit NaCl-Lsg. gewaschen. Nach Trocknung über MgSO₄ wird eingeengt und mit Flashchromatographie gereinigt. Ausbeute 3.47g (89%) **5** als amorpher Feststoff. DC (n- Heptan/ Ethylacetat 4:1). R_{f} =0.5, Edukt **4**_R_{f} = 0.6. C₂₃H₂₈FNO₂S (401.55). MS (M+H)⁺ = 402.2.

### Synthese von Verbindung 6:

3.47g (8.6 mmol) **5** werden in 24 ml Nitriersäure (aus 14 ml HNO₃ und 10 ml H₂SO₄) gelöst. Die Reaktionstemperatur wird durch Kühlen auf 20°C gehalten. Nach 30 Minuten gießt man die Lösung auf eine Mischung aus 700g Eis und 200 ml Ethylacetat. Die wässrige Phase wird abgetrennt und vorsichtig viermal mit 150 ml gesättigter NaHCO₃- Lsg. gewaschen. Dann wird über MgSO₄ getrocknet, eingeengt und mit Flashchromatographie gereinigt. Ausbeute 3.0g ( 78%) **6** als amorpher Feststoff. DC ( n- Heptan/ Ethylacetat 4:1). R_{f} = 0.4. C₂₃H₂₇N₂O₄SF (446.54). MS (M+H)⁺ = 447.2.

### Synthese von Verbindung 7:

3.0g (6.7 mmol) **6** werden in 50 ml 33 % igen HNMe₂ in Ethanol (Fluka) gelöst und eine Std. bei 50°C gerührt. Danach läßt man auf RT abkühlen und filtriert das entstandene Produkt. Ausbeute 2.86g (90%) **7** gelblich Kristalle Schmp. 188°C. DC ( n- Heptan/ Ethylacetat 2:1). R_{f} = 0.5, Edukt **7** R_{f} = 0.6. C₅₂H₃₃N₃O₄S (471.62). MS (M+H)⁺ =472.3.

### Synthese von Verbindung 8a/b als Enatiomerengemisch::

1.05g (2.2 mmol) **7** werden in 30 ml Toluol suspendiert und 500 mg Platin auf Aktivkohle (10% ig) zugegeben. Es wird 30 Std. bei 150 bar Wasserstoffdruck und 100°C im Schüttelautoklav hydriert. Zur Aufarbeitung filtriert man über Kieselgel, wäscht mit 100 ml Methanol nach, engt ein und reinigt den Rückstand mit Flashchromatographie. Ausbeute 495 mg (48%) **8a/b** als amorpher Feststoff. DC (n-Heptan/ Ethylacetat 1:1). R_{f} =0.3. C₂₅H₃₇N₃O₂S (443.65). MS (M+H)⁺ = 444.3.

### Synthese von Verbindung 10a/b als Diastereomerengemisch:

80 mg (0.18 mmol) **8a/b** und 100 mg (0.24 mmol) Penta-O-acetyl-D-gluconsäure (Org. Synth. Band 5, 887) werden in 4 ml DMF (Dimethylformamid) gelöst. Nacheinander werden dazu 100 mg (0.3 mmol) TOTU (Fluka), 35 mg (0.24 mmol) Oxim ( Hydroxyimino-cyanessigsäure-ethylester; Fluka) und 0.1 ml (0.78 mmol) NEM (4-Ethyl-morpholin) zugegeben. Nach einer Stunde bei Raumtemperatur wird mit 20 ml Ethylacetat verdünnt und dreimal mit Wasser gewaschen. Die organische Phase wird über MgSO₄ getrocknet, filtriert und eingeengt. Der Rückstand wird mittels Flashchromatographie (Ethylacetat/ n-Heptan 2:1) gereinigt und man erhält 130 mg (86%) **10a/b** als amorpher Feststoff. DC (Ethylacetat/ n-Heptan 2:1) R_{F}= 0.3. Das Produkt **10a/b** hat die gleiche Retention wie das Edukt **8a/b,** färbt allerdings mit 2 M Schwefelsäure unterschiedlich. C₄₁H₅₇N₃O₁₃S (131.97), MS (M + H)⁺ = 832.6.

### Synthese von Verbindung 11a/b als Diastereomerengemisch:

130 mg (0.16 mmol) **10a/b** werden in 5 ml Methanol gelöst. Nach Zugabe von 0.2 ml einer methanolischen 1 M Natriummethanolat- Lösung läßt man eine Stunde bei Raumtemperatur stehen. Dann wird mit methanolischer HCI- Lösung neutralisiert und eingeengt. Der Rückstand wird mit Flashchromatographie (Methylenchlorid/Methanol/ konz. Ammoniak 30/10/3) gereinigt und man erhält 78 mg (80%) **10a/b** als amorpher Feststoff. DC (Methylenchorid/ Methanol/ konz. Ammoniak 30/10/3). R_{f} = 0.4. C₃₁H₄₇N₃O₈S (621.80). MS (M + H)⁺ = 622.4.

### Synthese von Verbindung 12a/b als Diastereomerengemisch:

618 mg (0.74 mmol) **10a/b** werden in 30 ml Methylenchlorid gelöst und 385 mg (2.23 mmol) 70 % ige m-Chlorperbenzoesäure (Fluka) zugegeben. Nach 30 Minuten bei Raumtemperatur wird mit 100 ml Ethylacetat verdünnt und dreimal mit NaHCO₃-Lsg. gewaschen. Nach Trocknen mit MgSO₄ wird eingeengt und man erhält 700 mg Rohprodukt. Dieses Rohprodukt wird in 28 ml einer 0.05 M TiCl₄/ Acetonitril- Lsg. gelöst. Nach Zugabe von 300 mg festem Nal läßt man 15 Minuten rühren. Zur Aufarbeitung wird mit 150 ml Ethylacetat verdünnt und mit 100 ml 2 M Natriumthiosulfat- Lösung gewaschen. Die organische Phase wird über MgSO₄ getrocknet, eingeengt und der Rückstand mit Flashchromatographie gereinigt. Ausbeute 550 mg (87% über 2 Stufen) **12a/b** als amorpher Feststoff. DC (n-Heptan/Ethylacetat 1:2). R_{f}=0.3, Edukt **10a/b** R_{f}=0.35. C₄₁H₅₇N₃O₁₄S (847.99). MS (M+H)⁺ = 848.5.

### Synthese von Verbindung 13a/b als Diastereomerengemisch:

550 mg (0.65 mmol) **12a/b** werden in 20 ml Methanol gelöst. Nach Zugabe von 0.3 ml einer methanolischen 1 M Natriummethanolat- Lösung läßt man eine Stunde bei Raumtemperatur stehen. Dann wird mit methanolischer HCI- Lösung neutralisiert und eingeengt. Der Rückstand wird mit Flashchromatographie (Methylenchlorid/Methanol/ konz. Ammoniak 30/10/3) gereinigt und man erhält 370 mg (89%) **13a/b** als amorpher Feststoff. DC (Methylenchorid/ Methanol/ konz. Ammoniak 30/10/3). R_{f} = 0.4. C₃₁H₄₇N₃O₉S (637.80). MS (M + H)⁺ = 638.4.

### Synthese von Verbindung 15a/b als Diastereomerengemisch:

719 mg (1.6 mmol) **8a/b** werden in 30 ml Methylenchlorid und 2 ml Triethylamin gelöst. Zu dieser Lösung tropft man 0.5 ml (3.7 mmol) **14** und läßt 15 Minuten bei Raumtemperatur stehen. Die Lösung wird anschließend über Kieselgel filtriert und mit 100 ml Ethylacetat nachgewaschen. Nach dem Einengen wird der Rückstand mit Flashchromatographie gereinigt. Ausbeute 950 mg (95%) **15a/b** als amorpher Feststoff. DC (n-Heptan/Ethylacetat 1:1). R_{f} = 0.4. C₃₀H₄₄BrN₃O₃S (606.67). MS (M+H)⁺ = 607.3.

### Synthese von Verbindung 17a/b als Diastereomerengemisch:

897mg (1.47 mmol) **15a/b** werden in 20 ml DMF gelöst. Nach Zugabe von 1.3 g (7.1 mmol) **16** (Glucamin, Fluka) wird zwei Stunden auf 80°C erwärmt. Danach wird mit 50 ml Ethylacetat verdünnt und dreimal mit Wasser gewaschen. Die organische Phase wird über MgSO₄ getrocknet, filtriert und eingeengt. Der Rückstand wird mittels Flashchromatographie (Methylenchlorid/ Methanol/ konz. Ammoniak 30/10/3) gereinigt und man erhält 700 mg (67%) **17a/b** als amorphen Feststoff. DC (Methylenchlorid/ Methanol/ konz. Ammoniak 30/10/3). R_{f}= 0.4. C₃₆H₅₈N₄O₈S (706.95). MS (M + H)⁺ = 707.4.

### Synthese von Verbindung 19:

8.0 g (18.8 mmol) **9** (Penta-O-acetyl-D-gluconsäurechlorid; Org. Synth. Band 5, 887) werden zu einer Suspension von 8.0 g (40 mmol) **18** (Fluka) in 150 ml wasserfreiem DMF zugegeben. Diese Suspension wird 20 Stunden bei Raumtemperatur kräftig gerührt. Anschließend werden 500 ml Ethylacetat und 200 ml Wasser zugegeben. Die wässrige Phase wird nochmals mit 250 ml Ethylacetat extrahiert. Die vereinigte organische Phase wird dreimal mit Natriumchloridlösung gewaschen, über MgSO₄ getrocknet, filtriert und eingeengt. Ausbeute 9.5 g (86%) **19** als farbloses Öl. DC (Methylenchlorid/ Methanol konz. Ammoniak 30/10/ 3). R_{f}= 0.8. C₂₇H₄₃NO₁₃ (589.64). MS (M + H)⁺ = 590.4.

### Synthese von Verbindung 21a/b als Diastereomerengemisch:

200 mg (0.34 mmol) **19**, 70 mg (0.17mmol) **20a/b (20a/b** wird analog **8a/b** dargestellt, indem man mit 2-Butyl-2-ethyl-aziridin (R.Gauthier et al., J. Organomet. Chem. 140 **(1977)** 245 - 255) und **1** die Reaktionssequenz von Formelschema 1 durchführt), 240 mg TOTU, 80 mg Oxim und 0.3 ml NEM werden in 4 ml DMF analog der Vorschrift für Verbindung **11a/b** umgesetzt. Nach Flashchromatographie (Methylenchlorid/ Methanol konz. Ammoniak 30/ 5/ 1) erhält man 60 mg (46%, über zwei Stufen) **21a/b** als amorpher Feststoff. DC (Methylenchlorid/ Methanol/ konz. Ammoniak 30/ 5/ 1). R_{f}= 0.2. C₄₀H₆₄N₄O₉S (777.04). MS (M + H)⁺ = 777.8.

## Patentansprüche

1. Verbindungen der Formel I, 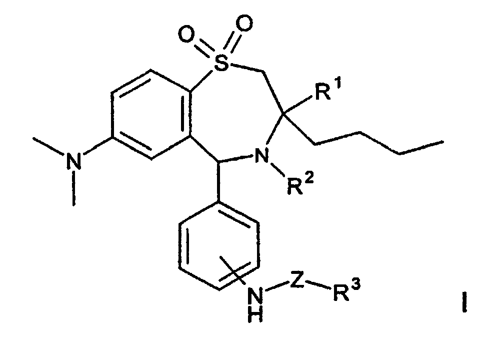 worin bedeuten
R¹ Methyl, Ethyl, Propyl, Butyl;
R² H, OH;
R³ Zuckerrest, Dizuckerrest, Trizuckerrest, Tetrazuckerrest,, wobei der Zuckerrest, Dizuckerrest, Trizuckerrest oder Tetrazuckerrest gegebenenfalls ein oder mehrfach substituiert ist durch eine Zucker-Schutzgruppe;
Z -(C=O)ₙ-C₀-C₁₆-Alkyl-, -(C=O)ₙ-C₀-C₁₆-Alkyl-NH-, -(C=O)ₙ-C₀-C₁₆-Alkyl-O-, -(C=O)ₙ-C₁-C₁₆-Alkyl-(C=O)ₘ, eine kovalente Bindung;
n 0 oder 1;
m 0 oder 1;
sowie deren pharmazeutisch verträglichen Salze und physiologisch funktionelle Derivate.

2. Verbindungen der Formel I, gemäß Anspruch 1, **dadurch gekennzeichnet**, das einer oder mehrere der Reste die folgende Bedeutung hat bzw. haben:
R¹ Ethyl, Propyl, Butyl;
R² H, OH;
R³ Zuckerrest, Dizuckerrest, wobei der Zuckerrest oder Dizuckerrest, gegebenenfalls ein oder mehrfach substituiert ist durch eine Zucker-Schutzgruppe;
Z -(C=O)ₙ-C₀-C₁₆-Alkyl-, -(C=O)ₙ-C₀-C₁₆-Alkyl-NH-, -(C=O)ₙ-C₀-C₁₆-Alkyl-O-,-(C=O)ₙ-C₁-C₁₆-Alkyl-(C=O)ₘ, einekovalente Bindung;
n 0 oder 1;
m 0 oder 1;
sowie deren pharmazeutisch verträglichen Salze.

3. Verbindungen der Formel I, gemäß Anspruch 1 oder 2, **dadurch gekennzeichnet, daß** einer oder mehrere der Reste die folgende Bedeutung hat bzw. haben:
R¹ Ethyl, Butyl;
R² H, OH;
R³ Zuckerrest, wobei der Zuckerrest gegebenenfalls ein oder mehrfach substituiert ist durch eine Zucker-Schutzgruppe;
Z -(C=O)-C₀-C₄-Alkyl, eine kovalente Bindung;
sowie deren pharmazeutisch verträglichen Salze.

4. Verfahren zur Herstellung von Verbindungen der Formel I, gemäß einem oder mehreren der Ansprüche 1 bis 3, **dadurch gekennzeichnet, daß** man nach folgendem Formelschema 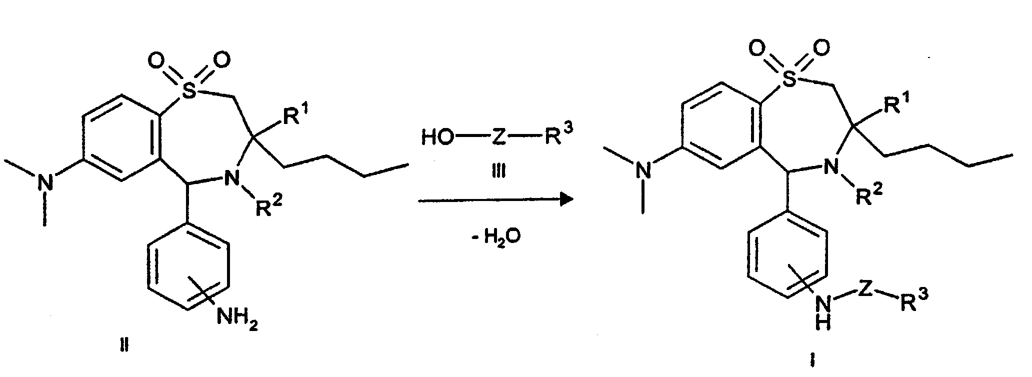 ein Amin der Formel II, in der R¹, R² und R³ die zu Formel I angegebenen Bedeutungen haben, mit einer Verbindung der Formel III, in der R³ und Z die zu Formel I angegebenen Bedeutungen haben, unter Wasserabspaltung zu einer Verbindung der Formel I umsetzt und die erhaltene Verbindung der Formel I gegebenenfalls in ein physiologisch verträgliche Salz oder ein physiologisch funktionelles Derivat überführt.

5. Arzneimittel enthaltend eine oder mehrere der Verbindungen gemäß einem oder mehreren der Ansprüche 1 bis 3.

6. Arzneimittel enthaltend eine oder mehrere der Verbindungen gemäß einem oder mehreren der Ansprüche 1 bis 3 und ein oder mehrere Statine.

7. Verbindungen gemäß einem oder mehreren der Ansprüche 1 bis 3 zur Anwendung als Medikament zur Behandlung von Lipidstoffwechsefstörungen.

8. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen gemäß einem oder mehreren der Ansprüche 1 bis 3 zur Herstellung eines Medikaments zur Behandlung von Hyperlipidämie.

10. Verwendung der Verbindungen gemäß einem oder mehreren der Ansprüche 1 bis 3 zur Herstellung eines Medikaments zur Beeinflussung des Serumcholesterinspiegels.

11. Verwendung der Verbindungen gemäß einem oder mehreren der Ansprüche 1 bis 3 zur Herstellung eines Medikaments zur Prävention arteriosklerotischer Erscheinungen.

## Claims

1. A compound of the formula I, 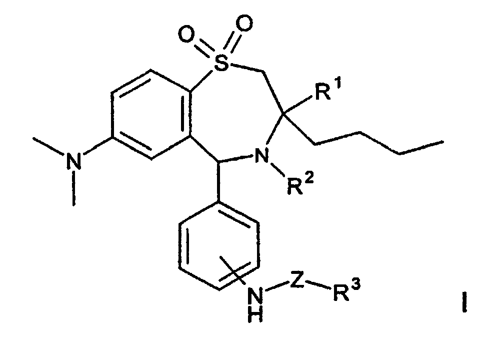 in which
R¹ is methyl, ethyl, propyl, butyl;
R² is H, OH;
R³ is a sugar residue, disugar residue, trisugar residue, tetrasugar residue, the sugar residue, disugar residue, trisugar residue or tetrasugar residue optionally being mono- or polysubstituted by a sugar protective group;
Z is -(C=O)ₙ-C₀-C₁₆-alkyl-, -(C=O)ₙ-C₀-C₁₆-alkyl-NH-, -(C=O)ₙ-C₀-C₁₆-alkyl-O-, -(C=O)ₙ-C₁-C₁₆-alkyl-(C=O)ₘ, a covalent bond;
n is 0 or 1;
m is 0 or 1;
or its pharmaceutically tolerable salts and physiologically functional derivatives.

2. A compound of the formula I as claimed in claim 1, wherein one or more of the radicals has or have the following meaning:
R¹ is ethyl, propyl, butyl;
R² is H, OH;
R³ is a sugar residue, disugar residue, the sugar residue or disugar residue optionally being mono- or polysubstituted by a sugar protective group;
Z is -(C=O)ₙ-C₀-C₁₆-alkyl-, -(C=O)ₙ-C₀-C₁₆-alkyl-NH-, -(C=O)ₙ-C₀-C₁₆-alkyl-O-, -(C=O)ₙ-C₁-C₁₆-alkyl-(C=O)ₘ, a covalent bond;
n is 0 or 1;
m is 0 or 1;
or its pharmaceutically tolerable salts.

3. A compound of the formula I as claimed in claim 1 or 2, wherein one or more of the radicals has or have the following meaning:
R¹ is ethyl, butyl;
R² is H, OH;
R³ is a sugar residue, the sugar residue optionally being mono- or polysubstituted by a sugar protective group;
Z is -(C=O)-C₀-C₄-alkyl, a covalent bond;
or its pharmaceutically tolerable salts.

4. A process for the preparation of compounds of the formula I as claimed in one or more of claims 1 to 3, which comprises reacting, according to the following reaction scheme 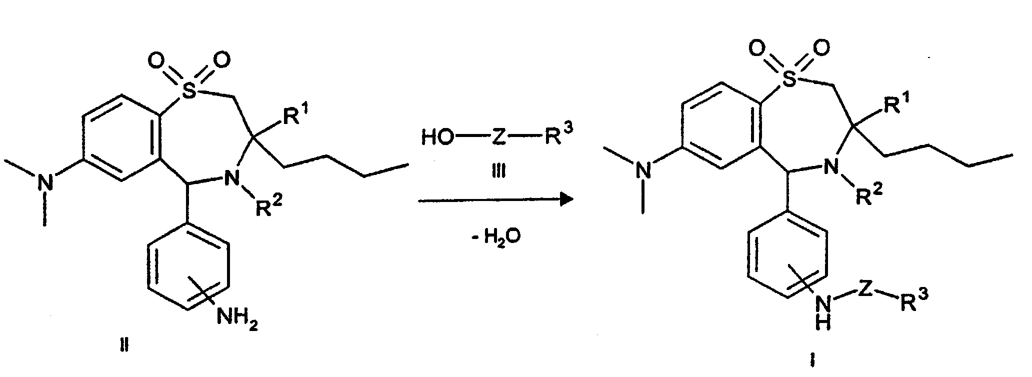 an amine of the formula II, in which R¹, R² and R³ have the meanings indicated for formula I, with a compound of the formula III, in which R³ and Z have the meanings indicated for formula I, with removal of water to give a compound of the formula I and optionally converting the compound of the formula I obtained into a physiologically tolerable salt or a physiologically functional derivative.

5. A pharmaceutical comprising one or more of the compounds as claimed in one or more of claims 1 to 3.

6. A pharmaceutical comprising one or more of the compounds as claimed in one or more of claims 1 to 3 and one or more statins.

7. A compound as claimed in one or more of claims 1 to 3 for use as a medicament for the treatment of lipid metabolism disorders.

8. A process for the production of a pharmaceutical comprising one or more of the compounds as claimed in one or more of claims 1 to 3, which comprises mixing the active compound with a pharmaceutically suitable carrier and bringing this mixture into a form suitable for administration.

9. Use of the compounds as claimed in one or more of claims 1 to 3 for the production of a medicament for treating hyperlipidemia.

10. The use of the compounds as claimed in one or more of claims 1 to 3 for the production of a medicament for influencing the serum cholesterol level.

11. The use of the compounds as claimed in one or more of claims 1 to 3 for the production of a medicament for preventing arteriosclerotic symptoms.

## Revendications

1. Composés de formule I, 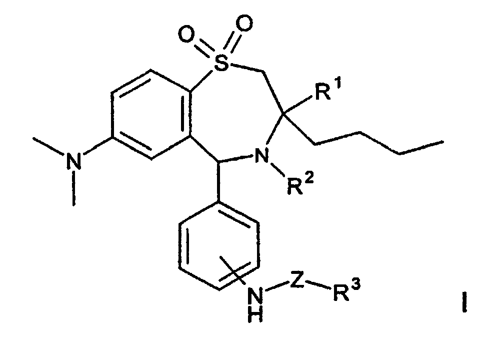 dans laquelle
R¹ représente un groupe méthyle, éthyle, propyle, butyle ;
R² représente H, OH ;
R³ représente un reste sucre, un reste disucre, un reste trisucre, un reste tétrasucre, où le reste sucre, le reste disucre, le reste trisucre ou le reste tétrasucre est éventuellement substitué une ou plusieurs fois par un groupe protecteur de sucre ;
Z -(C=O)ₙ-C₀-C₁₆-alkyl-, - (C=O) ₙ-C₀-C₁₆-alkyl-NH-, -(C=O)ₙ-C₀-C₁₆-alkyl-O-, -(C=O)ₙ-C₁-C₁₆-alkyl-(C=O)ₘ, une liaison covalente ;
n vaut 0 ou 1 ;
m vaut 0 ou 1 ;
ainsi que leurs sels pharmaceutiquement acceptables et leurs dérivés physiologiquement fonctionnels.

2. Composés de formule I, selon la revendication 1, **caractérisés en ce que** un ou plusieurs des restes possède ou possèdent la signification suivante :
R¹ représente un groupe éthyle, propyle, butyle ;
R² représente H, OH ;
R³ représente un reste sucre, un reste disucre, où le reste sucre ou le reste disucre est éventuellement substitué une ou plusieurs fois par un groupe protecteur de sucre ;
Z - (C=O)ₙ-C₀-C₁₆-alkyl-, - (C=O)ₙ-C₀-C₁₆-alkyl-NH-, -(C=O)ₙ-C₀-C₁₆-alkyl-O-, -(C=O)ₙ-C₁-C₁₆-alkyl-(C=O)ₘ, une liaison covalente ;
n vaut 0 ou 1 ;
m vaut 0 ou 1 ;
ainsi que leurs sels pharmaceutiquement acceptables.

3. Composés de formule I, selon la revendication 1 ou 2, **caractérisés en ce que** un ou plusieurs des restes possède ou possèdent la signification suivante:
R¹ représente un groupe éthyle, butyle ;
R² représente H, OH ;
R³ représente un reste sucre, où le reste sucre est éventuellement substitué une ou plusieurs fois par un groupe protecteur de sucre ;
Z -(C=O)-C₀-C₄-alkyl-, une liaison covalente ;
ainsi que leurs sels pharmaceutiquement acceptables.

4. Procédé pour la préparation de composés de formule I, selon une ou plusieurs des revendications 1 à 3, **caractérisé en ce qu'**on fait réagir selon le schéma réactionnel suivant 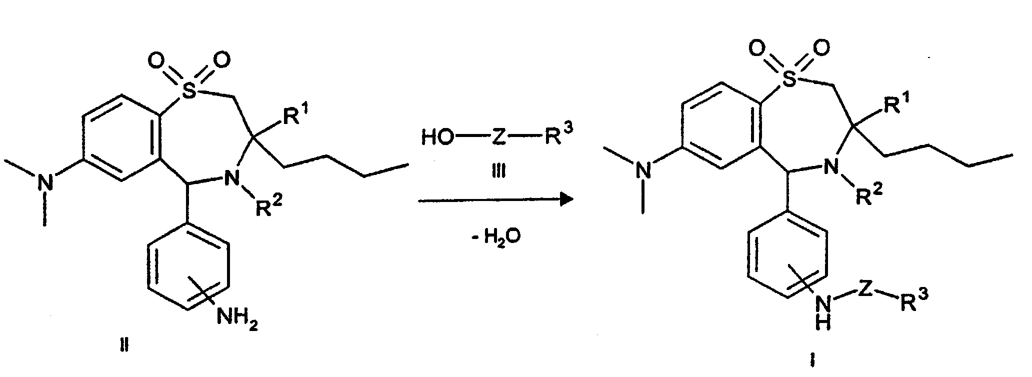 une amine de formule II, dans laquelle R¹, R² et R³ possèdent les significations indiquées à la formule I, sur un composé de formule III, dans laquelle R³ et Z ont les significations indiquées à la formule I, par élimination d'eau en un composé de formule I et on transforme le composé de formule I obtenu éventuellement en un sel physiologiquement acceptable ou un dérivé physiologiquement fonctionnel.

5. Médicament contenant un ou plusieurs des composés selon une ou plusieurs des revendications 1 à 3.

6. Médicament contenant un ou plusieurs des composés selon une ou plusieurs des revendications 1 à 3 et une ou plusieurs statines.

7. Composés selon une ou plusieurs des revendications 1 à 3 pour l'utilisation comme médicament pour le traitement des troubles du métabolisme lipidique.

8. Procédé pour la préparation d'un médicament contenant un ou plusieurs des composés selon une ou plusieurs des revendications 1 à 3, **caractérisé en ce que** l'on mélange le principe actif avec un véhicule pharmaceutiquement approprié et **en ce que** l'on met ce mélange en forme appropriée pour l'administration.

9. Utilisation des composés selon une ou plusieurs des revendications 1 à 3 pour la préparation d'un médicament pour le traitement de l'hyperlipidémie.

10. Utilisation des composés selon une ou plusieurs des revendications 1 à 3 pour la préparation d'un médicament pour influencer le taux de cholestérol sérique.

11. Utilisation des composés selon une ou plusieurs des revendications 1 à 3 pour la préparation d'un médicament pour la prévention des symptômes artériosclérotiques.
